# EUROPEAN PATENT SPECIFICATION

(11) **EP 1 962 757 B1**
(45) Date of publication and mention of the grant of the patent: **19.05.2010**
(21) Application number: 06838823.0
(22) Date of filing: 30.11.2006
(51) Int. Cl.: A61F 13/42

(54) **GARMENTS WITH EASY-TO-USE SIGNALING DEVICE**
KLEIDUNGSSTÜCKE MIT EINFACH ANZUWENDENDER SIGNALVORRICHTUNG
VETEMENTS AVEC DISPOSITIF DE SIGNALISATION SIMPLE D'UTILISATION

(30) Priority: 15.12.2005 US 303283
(43) Date of publication of application: 03.09.2008
(73) Proprietor: KIMBERLY-CLARK WORLDWIDE, INC., Neenah, WI 54956 (US)
(72) Inventor: LONG, Andrew. M., Appleton, Wisconsin 54915 (US); TIPPEY, Darold D., Neenah, Wisconsin 54956 (US)
(74) Representative: Mabey, Katherine Frances
(86) International application number: PCT/US2006/046072
(87) International publication number: WO 2007/070267

(56) References cited:
- US-A- 5 808 554
- US-B1- 6 200 250

## Description

### BACKGROUND OF THE INVENTION

Absorbent articles such as diapers, training pants, incontinence products, feminine hygiene products, swim undergarments, and the like conventionally include a liquid permeable body-side liner, a liquid impermeable outer cover, and an absorbent core. The absorbent core is typically located in between the outer cover and the liner for taking in and retaining liquids (e.g., urine) exuded by the wearer.

The absorbent core can be made of, for instance, superabsorbent particles. Many absorbent particles, especially super absorbent particles, are so efficient at absorbing liquids that it is sometimes difficult to tell whether or not the absorbent article has been insulted with a body fluid.

Accordingly, various types of moisture or wetness indicators have been suggested for use in absorbent articles. The wetness indicators may include alarm devices that are designed to assist parents or attendants to identify a wet diaper condition quickly upon insult. The devices produce either a visual or an audible signal.

In some embodiments, for instance, inexpensive conductive threads or foils have been placed in the absorbent articles. The conductive materials serve as conductive leads for a signaling device and form an open circuit in the article that can be closed when a body fluid, such as urine, closes the circuit. In these embodiments, although the absorbent articles may be disposable, the signaling devices are not. Thus, the signaling devices are intended to be removed from the article and reattached to a subsequent article.

U.S. 5,808,554 discloses a moisture detecting liner for a diaper, and a process for manufacture thereof.

Problems, however, have been encountered in designing an attachment mechanism for the signaling device that allows for a very reliable attachment of the signaling device to the conductive leads but does not appreciably increase the cost of the absorbent article. Further, problems have been experienced in designing an attachment mechanism that can be incorporated into the absorbent article during high speed manufacturing processes.

The present invention provides a garment in accordance with claim 1.

In general, the present disclosure is directed to garments with easy-to-use signaling devices. The signaling device, for instance, may be configured to indicate to a user that a body fluid is present in the absorbent article. For example, in one embodiment, the garment includes a chassis including an outer cover having an interior surface and an exterior surface. The chassis includes a crotch region positioned in between a front region and a back region. The front region and the back region jointly define a waist region. The garment also includes first and second conductive elements contained in the chassis. The conductive elements being located in at least the waist region. The first and second conductive elements form part of a circuit. The garment includes a signaling device including at least one first terminal and at least one second terminal. The first and second terminals are adapted to operatively connect to the first and second conductive elements. Further, the first and second terminals operatively connect to the first and second conductive elements through at least one orifice in the outer cover.

In embodiments, the first terminal comprises a terminal top and a terminal stalk. The terminal top has a terminal top width. The terminal stalk has a terminal stalk width. The terminal top width being greater than the terminal stalk width.

### BRIER DESCRIPTION OF THE DRAWINGS

The foregoing and other features and aspects of the present invention and the manner of attaining them will become more apparent, and the invention itself will be better understood by reference to the following description, appended claims and accompanying drawings, where:
Figure 1 is a rear perspective view of one embodiment of an absorbent article;
Figure 2 is a front perspective view of the absorbent article illustrated in Figure 1;
Figure 3 is a plan view of the absorbent article illustrated in Figure 1 with the article in an unfastened, unfolded and laid flat condition showing the surface of the article that faces away from the wearer;
Figure 4 is a plan view similar to Figure 3 illustrating the surface of the absorbent article that faces the wearer when worn and with portions cut away to show underlying features;
Figure 5 is a perspective view of the embodiment shown in Figure 1 further including one embodiment of a signaling device;
Figure 6 is a plan view of another embodiment of an absorbent article illustrating the article in an unfastened, unfolded and laid flat condition illustrating the surface of the article that faces away from the wearer;
Figure 7 is a perspective view of one embodiment of an attachment mechanism for attaching a signaling device to the absorbent article;
Figure 7A is cross section of the absorbent article of Figure 7 as viewed along line 7A-7A;
Figure 8 is another embodiment of an attachment mechanism for attaching a signaling device to an absorbent article;
Figure 9 is still another embodiment of an attachment mechanism for attaching a signaling device to an absorbent article;
Figure 10 is a cross section view of another embodiment of an attachment mechanism for attaching a signaling device to an absorbent article;
Figure 11 is a cross section view of another embodiment of an attachment mechanism for attaching a signaling device to an absorbent article;
Figure 12 is a enlarged cross section view of the embodiment illustrated in Figure 11; and
Figure 13 is a perspective view of another embodiment of an attachment mechanism for attaching a signaling device to an absorbent article.

Repeat use of reference characters in the present specification and drawings is intended to represent the same or analogous features or elements of the present invention.

### DETAILED DESCRIPTION OF THE INVENTION

It is to be understood by one of ordinary skill in the art that the present discussion is a description of exemplary embodiments only, and is not intended as limiting the broader aspects of the present invention.

The present disclosure is generally directed to absorbent articles adapted to be attached to a signaling device that may be configured to indicate the presence of a body fluid in the absorbent article or other changes in the condition of the product or wearer. The absorbent article may be, for instance, a diaper, a training pant, an incontinence product, a feminine hygiene product, a medical garment, a bandage, and the like. Absorbent articles may include an open circuit that becomes closed when a conductive fluid, such as a body fluid, is present in between a pair of conductive leads. Alternatively, absorbent articles may include a closed circuit that becomes open when a fluid, such as a body fluid, is present. Generally, the absorbent articles containing the circuit are disposable meaning that they are designed to be discarded after a limited use rather than being laundered or otherwise restored for reuse.

The circuit contained within the absorbent articles of the present disclosure is configured to be attached to a signaling device. The signaling device can provide power to the circuit while also including some type of audible, visible and/or electromagnetic signal that indicates to the user the presence of a body fluid. Although the absorbent article may itself be disposable, the signaling device may be reusable from article to article. In this regard, the present disclosure is particularly directed to different types of attachment mechanisms that allow easy connection between the circuit in the absorbent article and the signaling device.

As described above, the circuit in combination with the signaling device may be configured to indicate the presence of a body fluid contained within the absorbent article. The particular targeted body fluid may vary depending upon the particular type of absorbent article and the desired application. For instance, in one embodiment, the absorbent article comprises a diaper, a training pant, or the like and the signaling device is configured to indicate the presence of urine. Alternatively, the signaling device may be configured to indicate the presence of a metabolite that would indicate the presence of a diaper rash. For adult incontinence products and feminine hygiene products, on the other hand, the signaling device may be configured to indicate the presence of a yeast or of a particular constituent in urine or menses, such as a polysaccharide.

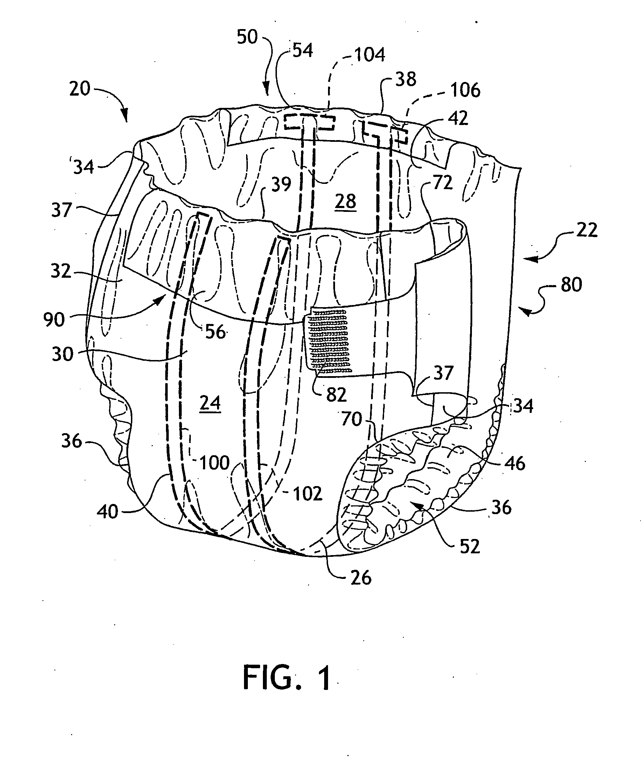

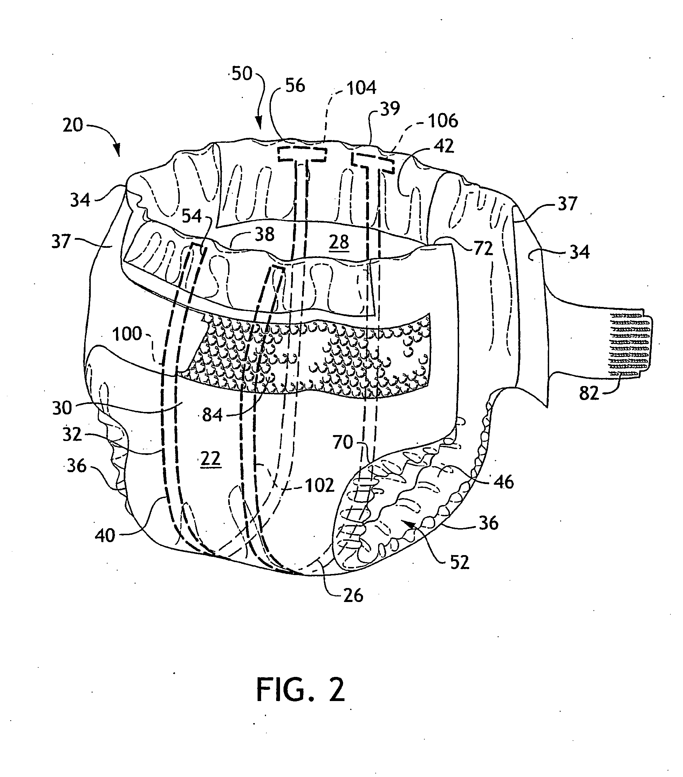

Referring to **Figs. 1** and **2**, for exemplary purposes, an absorbent article **20** is shown. The absorbent article **20** may or may not be disposable. It is understood that the present invention is suitable for use with various other absorbent articles intended for personal wear, including but not limited to diapers, training pants, swim pants, feminine hygiene products, incontinence products, medical garments, surgical pads and bandages, other personal care or health care garments, and the like without departing from the scope of the present invention.

By way of illustration only, various materials and methods for constructing absorbent articles such as the diaper **20** of the various aspects of the present invention are disclosed in U.S. Patent No. 4,798,603 issued January 17, 1989, to Meyer et al.; U.S. Patent No. 5,176,672 issued January 5, 1993, to Bruemmer et al., U.S. Patent No. 5,509,915 issued April 23, 1996 to Hanson et al., U.S. Patent No. 5,993,433 issued November 30, 1999 to St. Louis et al., and U.S. Patent No. 6,248,097 issued June 19, 2001 to Beitz et al., PCT Patent Application WO 00/37009 published June 29, 2000 by A. Fletcher et al; U.S. Patent 4,940,464 issued July 10, 1990 to Van Gompel et al.; U.S. Patent 5,766,389 issued June 16, 1998 to Brandon et al., and U.S. Patent 6,645,190 issued November 11, 2003 to Olson et al.

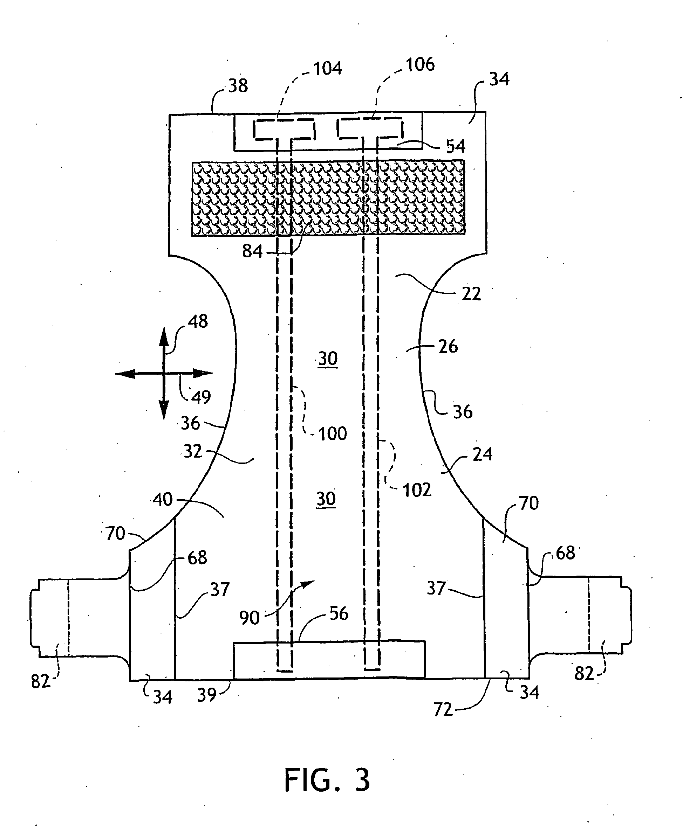

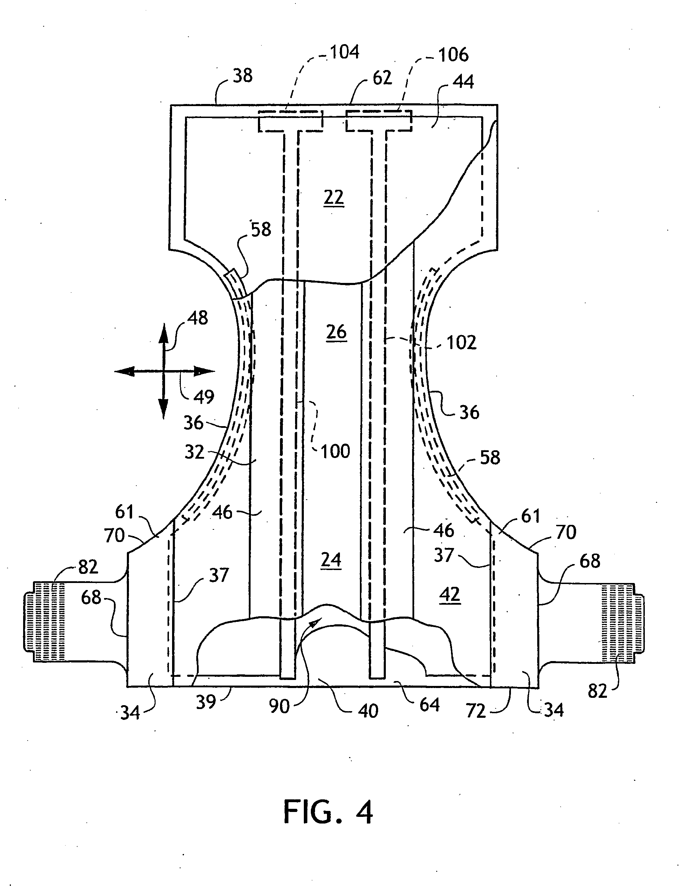

A diaper **20** is representatively illustrated in **Fig. 1** in a partially fastened condition. The diaper **20** shown in **Figs. 1** and **2** is also represented in **Figs. 3** and **4** in an opened and unfolded state. Specifically, **Fig. 3** is a plan view illustrating the exterior side of the diaper **20,** while **Fig. 4** illustrates the interior side of the diaper **20.** As shown in **Figs. 3** and **4****,** the diaper **20** defines a longitudinal direction **48** that extends from the front of the article when worn to the back of the article. Opposite to the longitudinal direction **48** is a lateral direction **49.**

The diaper **20** defines a pair of longitudinal end regions, otherwise referred to herein as a front region **22** and a back region **24,** and a center region, otherwise referred to herein as a crotch region **26,** extending longitudinally between and interconnecting the front and back regions **22, 24.** The diaper **20** also defines an inner surface **28** adapted in use (e.g., positioned relative to the other components of the article **20)** to be disposed toward the wearer, and an outer surface **30** opposite the inner surface. The front and back regions **22, 24** are those portions of the diaper **20,** which when worn, wholly or partially cover or encircle the waist or mid-lower torso of the wearer. The crotch region **26** generally is that portion of the diaper **20** which, when worn, is positioned between the legs of the wearer and covers the lower torso and crotch of the wearer. The absorbent article **20** has a pair of laterally opposite side edges **36** and a pair of longitudinally opposite waist edges, respectively designated front waist edge **38** and back waist edge **39.**

The illustrated diaper **20** includes a chassis **32** that, in this embodiment, encompasses the front region **22,** the back region **24,** and the crotch region **26.** Referring to **Figs. 1-4****,** the chassis **32** includes an outer cover **40** and a bodyside liner **42 (****Figs. 1** and **4****)** that may be joined to the outer cover **40** in a superimposed relation therewith by adhesives, ultrasonic bonds, thermal bonds or other conventional techniques. Referring to **Fig. 4****,** the liner **42** may suitably be joined to the outer cover **40** along the perimeter of the chassis **32** to form a front waist seam **62** and a back waist seam **64.** As shown in **Fig. 4****,** the liner **42** may suitably be joined to the outer cover **40** to form a pair of side seams **61** in the front region **22** and the back region **24.** The liner **42** can be generally adapted, i.e., positioned relative to the other components of the article **20,** to be disposed toward the wearer's skin during wear of the absorbent article. The chassis **32** may further include an absorbent structure **44** particularly shown in **Fig. 4** disposed between the outer cover **40** and the bodyside liner **42** for absorbing liquid body exudates exuded by the wearer, and may further include a pair of containment flaps **46** secured to the bodyside liner **42** for inhibiting the lateral flow of body exudates.

The elasticized containment flaps **46** as shown in **Fig. 4** define a partially unattached edge which assumes an upright configuration in at least the crotch region **26** of the diaper **20** to form a seal against the wearer's body. The containment flaps **46** can extend longitudinally along the entire length of the chassis **32** or may extend only partially along the length of the chassis. Suitable constructions and arrangements for the containment flaps **46** are generally well known to those skilled in the art and are described in U.S. Patent 4,704,116 issued November 3, 1987 to Enloe.

To further enhance containment and/or absorption of body exudates, the diaper **20** may also suitably include leg elastic members **58 (****Fig. 4**), as are known to those skilled in the art. The leg elastic members **58** can be operatively joined to the outer cover **40** and/or the bodyside liner **42** and positioned in the crotch region **26** of the absorbent article **20.**

The leg elastic members **58** can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic retractive forces are imparted to the substrate. In one particular aspect, for example, the leg elastic members **58** may include a plurality of dry-spun coalesced multifilament spandex elastomeric threads sold under the trade name LYCRA and available from Invista, Wilmington, Delaware, U.S.A.

In some embodiments, the absorbent article **20** may further include a surge management layer (not shown) which may be optionally located adjacent the absorbent structure **44** and attached to various components in the article **20** such as the absorbent structure **44** or the bodyside liner **42** by methods known in the art, such as by using an adhesive. A surge management layer helps to decelerate and diffuse surges or gushes of liquid that may be rapidly introduced into the absorbent structure of the article. Desirably, the surge management layer can rapidly accept and temporarily hold the liquid prior to releasing the liquid into the storage or retention portions of the absorbent structure. Examples of suitable surge management layers are described in U.S. Pat. No. 5,486,166; and U.S. Pat. No. 5,490,846. Other suitable surge management materials are described in U.S. Pat. No. 5,820,973.

As shown in **Figs. 1-4****,** the absorbent article **20** further includes a pair of opposing elastic side panels **34** that are attached to the back region of the chassis **32.** As shown particularly in **Figs. 1** and **2****,** the side panels **34** may be stretched around the waist and/or hips of a wearer in order to secure the garment in place. As shown in **Figs. 3** and **4****,** the elastic side panels are attached to the chassis along a pair of opposing longitudinal edges **37.** The side panels **34** may be attached or bonded to the chassis **32** using any suitable bonding technique. For instance, the side panels **34** may be joined to the chassis by adhesives, ultrasonic bonds, thermal bonds, or other conventional techniques.

In an alternative embodiment, the elastic side panels may also be integrally formed with the chassis **32.** For instance, the side panels **34** may comprise an extension of the bodyside liner **42,** of the outer cover **40,** or of both the bodyside liner **42** and the outer cover 40.

In the embodiments shown in the figures, the side panels **34** are connected to the back region of the absorbent article **20** and extend over the front region of the article when securing the article in place on a user. It should be understood, however, that the side panels **34** may alternatively be connected to the front region of the article **20** and extend over the back region when the article is donned.

With the absorbent article **20** in the fastened position as partially illustrated in **Figs. 1** and **2****,** the elastic side panels **34** may be connected by a fastening system **80** to define a 3-dimensional diaper configuration having a waist opening **50** and a pair of leg openings **52.** The waist opening **50** of the article **20** is defined by the waist edges **38** and **39** which encircle the waist of the wearer.

In the embodiments shown in the figures, the side panels are releasably attachable to the front region **22** of the article **20** by the fastening system. It should be understood, however, that in other embodiments the side panels may be permanently joined to the chassis **32** at each end. The side panels may be permanently bonded together, for instance, when forming a training pant or absorbent swimwear.

The elastic side panels **34** each have a longitudinal outer edge **68,** a leg end edge **70** disposed toward the longitudinal center of the diaper **20,** and waist end edges **72** disposed toward a longitudinal end of the absorbent article. The leg end edges **70** of the absorbent article **20** may be suitably curved and/or angled relative to the lateral direction **49** to provide a better fit around the wearer's legs. However, it is understood that only one of the leg end edges **70** may be curved or angled, such as the leg end edge of the back region **24,** or alternatively, neither of the leg end edges may be curved or angled, without departing from the scope of the present invention. As shown in **Fig. 4****,** the outer edges **68** are generally parallel to the longitudinal direction **48** while the waist end edges **72** are generally parallel to the transverse axis **49.** It should be understood, however, that in other embodiments the outer edges **68** and/or the waist edges **72** may be slanted or curved as desired. Ultimately, the side panels **34** are generally aligned with a waist region **90** of the chassis.

The fastening system **80** may include laterally opposite first fastening components **82** adapted for refastenable engagement to corresponding second fastening components **84.** In the embodiment shown in the figures, the first fastening component **82** is located on the elastic side panels **34,** while the second fastening component **84** is located on the front region **22** of the chassis **32.** In one aspect, a front or outer surface of each of the fastening components **82, 84** includes a plurality of engaging elements. The engaging elements of the first fastening components **82** are adapted to repeatedly engage and disengage corresponding engaging elements of the second fastening components **84** to releasably secure the article **20** in its three-dimensional configuration.

The fastening components **82, 84** may be any refastenable fasteners suitable for absorbent articles, such as adhesive fasteners, cohesive fasteners, mechanical fasteners, or the like. In particular aspects the fastening components include mechanical fastening elements for improved performance. Suitable mechanical fastening elements can be provided by interlocking geometric shaped materials, such as hooks, loops, bulbs, mushrooms, arrowheads, balls on stems, male and female mating components, buckles, snaps, or the like.

In the illustrated aspect, the first fastening components **82** include hook fasteners and the second fastening components **84** include complementary loop fasteners. Alternatively, the first fastening components **82** may include loop fasteners and the second fastening components **84** may be complementary hook fasteners. In another aspect, the fastening components **82, 84** can be interlocking similar surface fasteners, or adhesive and cohesive fastening elements such as an adhesive fastener and an adhesive-receptive landing zone or material; or the like. One skilled in the art will recognize that the shape, density and polymer composition of the hooks and loops may be selected to obtain the desired level of engagement between the fastening components **82, 84.** Suitable fastening systems are also disclosed in the previously incorporated PCT Patent Application WO 00/37009 published June 29, 2000 by A. Fletcher et al. and U.S. Patent 6,645,190 issued November 11, 2003 to Olson et al.

In the embodiment shown in the figures, the fastening components **82** are attached to the side panels **34** along the edges **68.** In this embodiment, the fastening components **82** are not elastic or extendable. In other embodiments, however, the fastening components may be integral with the side panels **34.** For example, the fastening components may be directly attached to the side panels **34** on a surface thereof.

In addition to possibly having elastic side panels, the absorbent article **20** may include various waist elastic members for providing elasticity around the waist opening. For example, as shown in the figures, the absorbent article **20** can include a front waist elastic member **54** and/or a back waist elastic member **56.**

As described above, the present disclosure is particularly directed to incorporating a body fluid indicating system, such as a wetness indicating system into the absorbent article **20.** In this regard, as shown in **Figs. 1-4****,** the absorbent article **20** includes a first conductive element **100** spaced from a second conductive element **102.** In this embodiment, the conductive elements extend from the front region **22** of the absorbent article to the back region **24** without intersecting. The conductive elements **100** and **102** can comprise any suitable conductive material, such as a conductive thread or a conductive foil for example include 112-S silver metallic conductive paste (ink) from Electroscience Laboratories, Inc. and conductive foil described in U.S. Patent 6,417,455 issued July 9, 2002 to Zein et. Al. The first conductive element **100** may not intersect the second conductive element **102** in order to form an open circuit that may be closed, for instance, when a conductive fluid is positioned in between the conductive elements. In other embodiments, however, the first conductive element **100** and the second conductive element **102** may be connected to a sensor within the chassis. The sensor may be used to sense changes in temperature or may be used to sense the presence of a particular substance, such as a metabolite.

In the embodiment shown in **Fig. 1****,** the conductive elements **100** and **102** extend the entire length of the absorbent article **20.** It should be understood, however, that in other embodiments the conductive elements may extend only to the crotch region **26** or may extend to any particular place in the absorbent article where a body fluid is intended to be sensed.

The conductive elements **100** and **102** may be incorporated into the chassis **32** at any suitable location as long as the conductive elements are positioned so as to contact a body fluid that is absorbed by the absorbent article **20.** In this regard, the conductive elements **100** and **102** generally lie inside the outer cover **40.** In fact, in one embodiment, the conductive elements **100** and **102** may be attached or laminated to the inside surface of the outer cover **40** that faces the absorbent structure **44.** Alternatively, however, the conductive elements **100** and **102** may be positioned on the absorbent structure **44** or positioned on the liner **42.**

The conductive element **100** and **102** may be connected directly to a signaling device, either through direct or indirect contact. The first conductive element **100** may be attached to a first conductive pad member **104,** while the second conductive element **102** may be connected to a second conductive pad member **106.** The pad members **104** and **106** may be provided for making a reliable connection between the open circuit formed by the conductive elements to a signaling device that is intended to be installed on the chassis by the consumer or manufacturer. The pad members **104** and **106** may create a target zone for attaching the signaling device and the conductive leads or elements.

The conductive pad members **104** and **106** may have a relatively large surface area in relation to the conductive elements **100** and **102.** For example, the conductive pad members **104** and **106** may have a surface area of at least 1 cm², at least 2 cm², and, in one embodiment, at least 3 cm². For instance, in one embodiment, the surface area of each pad member may be from about 2 cm² to about 4 cm².

The position of the conductive pad members **104** and **106** on the absorbent article **20** can vary depending upon where it is desired to mount the signaling device. For instance, in **Figs. 1****,** **3** and **4****,** the conductive pad members **104** and **106** are positioned in the front region **22** along the waist opening of the article. In **Fig. 2****,** on the other hand, the conductive pad members **104** and **106** are positioned in the back region **24** along the waist opening of the article. It should be appreciated, however, that in other embodiments, the absorbent article **20** may include conductive pad members being positioned at each end of each conductive element **100** and **102.** In still other embodiments, it should be understood that the pad members may be located along the side of the article or towards the crotch region of the article.

The position of the conductive pad members **104** and **106** within the multiple layers of the chassis **32** may also vary depending upon where it is desired to connect the signaling device and the type of attachment mechanism used to make a connection with the signaling device. As described above, the pad members **104** and **106** are electrically connected to the conductive elements **100** and **102.** Thus, in one embodiment, the pad members **104** and **106** are positioned below (toward the body side) at least one layer of the outer cover **40.** Positioning the pad members **104** and **106** below at least one layer of material may provide various advantages in some embodiments. For instance, locating the pad members **104** and **106** below at least one layer of material within the chassis **32** protects the pad members during shipping and storage and from forming a short circuit during use especially if the pad members are located adjacent one another. Another benefit to placing the pad members under at least one layer of material is the ability to easily manufacture the absorbent article **20** at high machine speeds.

It should be understood, however, that in other embodiments the conductive pad members **104** and **106** may be positioned at an exterior surface of the chassis **32.** For instance, the pad members **104** and **106** may be positioned on the outside surface or on the inside surface as desired.

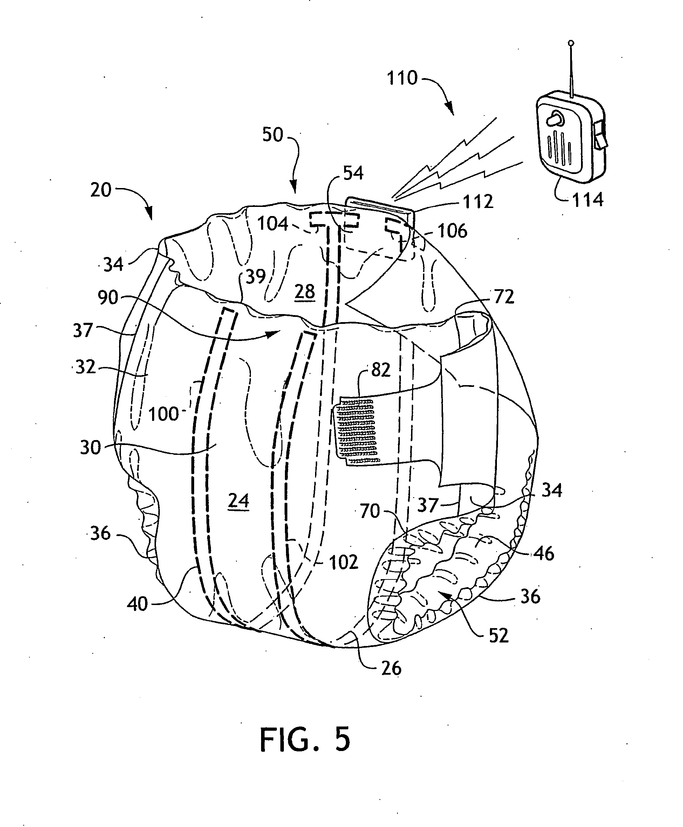

Referring to **Fig. 5****,** for exemplary purposes, a signaling device **110** (as depicted by ref. numerals **112** and **114)** is shown attached to the conductive pad members **104** and **106.** As shown, in this embodiment, the signaling device generally **110** includes a transmitter **112** and a receiver **114.** The transmitter **112** includes a pair of opposing terminals that are electrically connected to the corresponding conductive elements. When a body fluid is present in the absorbent article **20,** the open circuit formed by the conductive elements **100** and **102** is closed which, in turn, activates the signaling device **110.** In particular, in this embodiment, the transmitter **112** sends a wireless signal to the receiver **114** which then indicates to a user that a body fluid is present in the absorbent article.

The signaling device **110** can emit an audible signal or a visual signal in order to indicate to the user that the circuit has been closed. The audible signal, for instance, may be as simple as one or more beeps to perhaps emitting a musical tune. Similarly, if the signaling device **110** issues a visible signal, the visible signal may comprise a few lights or an interactive display. In still another embodiment, the receiver **114** of the signaling device **110** may be configured to vibrate when the circuit within the absorbent article is closed.

In the embodiment shown in **Fig. 5****,** the signaling device **110** includes a transmitter **112** in combination with a receiver **114.** It should also be understood, however, that the signaling device may comprise a single unit that remains attached to the absorbent article **20.** For example, the signaling device may be mounted on the absorbent article and issue a visible signal and/or an audible signal from the article itself.

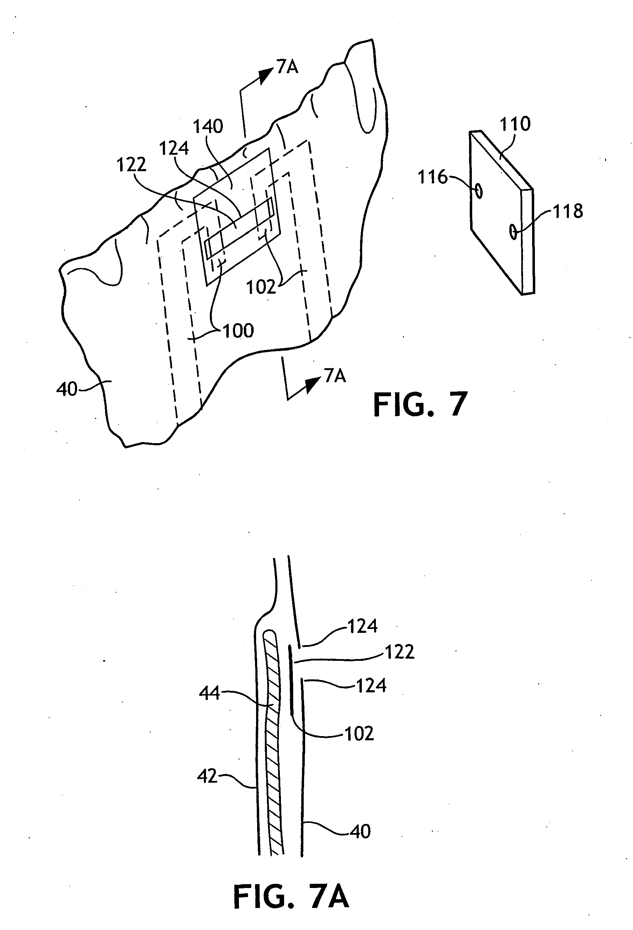

Attachment mechanisms between the conductive pad members or the conductive elements and the signaling device and the particular construction of the pad member for each embodiment will now be described in detail with respect to **Figs. 7-13****.** The signaling device **110** may connect to the to the chassis **32** in many different ways. The absorbent article **20** may include a signaling device receptacle **140** adapted to connect the signaling device **110** to the absorbent chassis **32.** The signaling device **110** may connect to the to the chassis utilizing adhesives, cohesives, magnets, hook and loop, clips or snaps. Alternatively, the signaling device **110** may connect to the chassis **140** by capturing all or portions of the signaling device **110** in a pocket or utilizing elastic portions which wrap around the signaling device **110** maintaining it in place.

The signaling device **110** is adapted to connect to the article **20** through an orifice **122** in the outer cover **40.** The design of the signaling device **110,** the chassis **32,** and receptacle **140** (if utilized) may be designed such that the first and second terminals **116, 118** operatively connect to the first and second conductive elements **100, 102** through one, two or more orifices **122.** This compatibility between the signaling device **110** and the chassis 32 may allow for easier, less complicated and higher quality of connection between the terminals **116, 118** and the conductive elements **100, 102.** This feature may be very advantageous when utilizing outer covers **40** which have high loft or may be very cloth like.

As shown in **Fig. 7****,** the first conductive element **100** is disposed in the left portion of the receptacle **140** and the second conductive element **102** is disposed in the right portion of the receptacle **140.** Also shown in **Fig. 7** is a signaling device **110** including a first terminal **116** and a second terminal **118.**

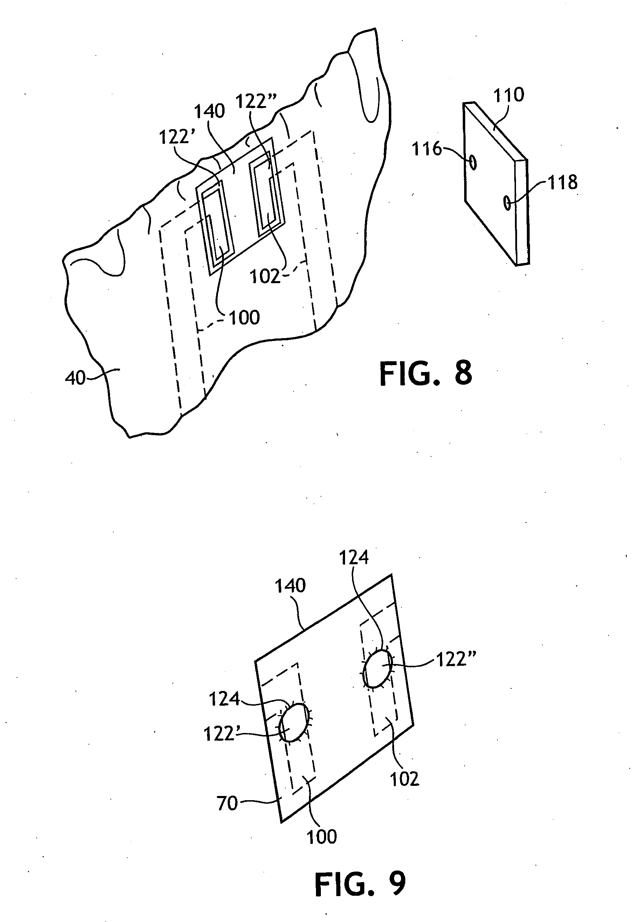

The first terminal **116** of the signaling device **110** contacts the first or second conductive element **100, 102** through at least one orifice **122** in the outer cover **40.** Further the second terminal **118** contacts the first or second conductive element **100, 102** through at least one orifice **122** in the outer cover **40.** As shown in **FIG. 7****,** the first and second terminal **116, 118** may contact the first and second conductive elements **100, 102** through a single orifice **122** in the outer. Alternatively, as shown in **FIG. 8** the first terminal **116** may contact the first or second conductive element **100, 102** through a first orifice **122'** and the second terminal **118** may contact the first or second conductive element **100, 102** through a second orifice **122".**

The orifice **122** may be defined by an orifice perimeter **124** that completely surrounds the orifice **122.** The orifice perimeter **124** may define an orifice area, which is the open area in the plane of the outer cover **40** surrounded by the orifice perimeter **124.** The orifice area may be any size dependent on the specific design of the chassis **32** and signaling device **110.** The orifice area may be smaller than, larger than or the same size as the area of the first or second terminal **116, 118.** The orifice area may be greater than the combination of the area of the first and second terminals **116, 118.** Further yet, the orifice area may be larger than or the same size of the area of the signaling device **110.**

The orifice **122, 122', 122"** may have any shape dependent upon the specific design of the chassis **32** and signaling device **110.** As shown in **FIG. 7****,** the orifice **122** may have the rectangular shape. As shown in **FIG. 9****,** the orifices **122', 122"** may have a circular shape.

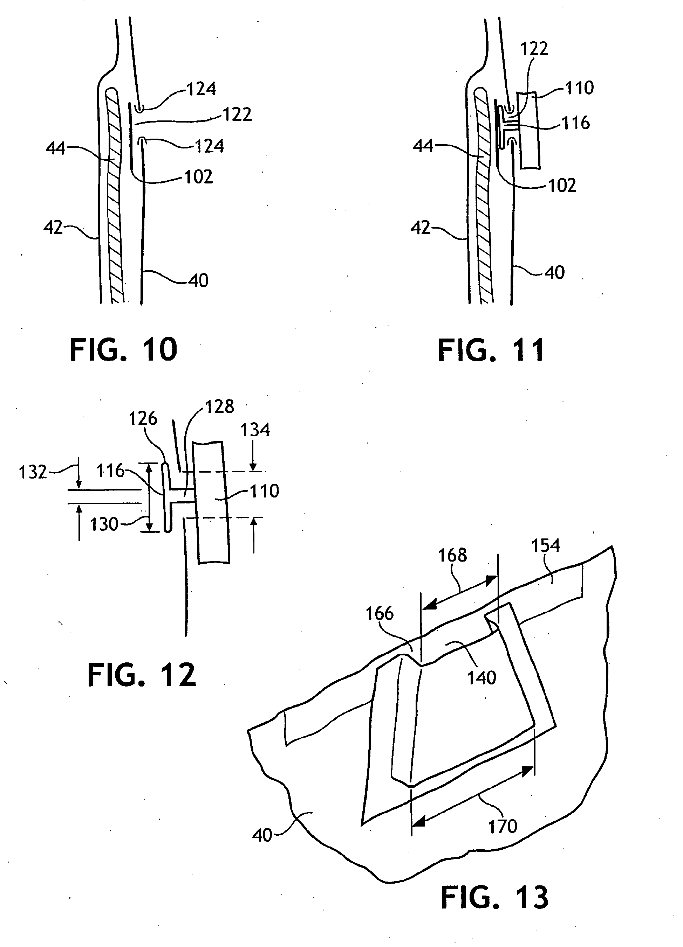

The orifice perimeter **124** may be formed by an edge of the outer cover **40,** as illustrated in **FIG. 7A****.** Alternatively, the orifice perimeter **124** may be formed by a second material added the edge of the outer cover **40,** for example, piping, as illustrated in **FIG. 10****.** The orifice perimeter **124** may be formed by a portion of the outer cover **40** which has been folded or rolled back upon itself.

The orifice perimeter **124** may include an attachment mechanism adapted to connect the chassis **32** to the signaling device **110.** These attachment mechanism may include adhesive fasteners, cohesive fasteners, mechanical fasteners, or the like. The fastening components may include mechanical fastening elements including hooks, loops, bulbs, mushrooms, arrowheads, balls on stems, male and female mating components, buckles, snaps or the like.

The orifice perimeter **124** is elastically stretchable. The stretchability in the orifice perimeter **124** may come from stretchability in the outer cover **40.** Alternatively, the stretchability in the orifice perimeter **124** may come from a second material added to the edge of the outer cover **40,** for example, an elastic piping material.

**FIGs. 11** and **12** illustrate a side view of a garment and a signaling device **110.** As illustrated, the signaling device includes a terminal **116** including a terminal top **126** and a terminal stalk **128.** The terminal stalk **128** extends outward from a main body of the signaling device **110.** The terminal top **126** is located at the distal end of the terminal stalk **128,** remoter from the main body of the signaling device **110.** The terminal top **126** has a terminal top width **130.** The terminal stalk **128** has a terminal stalk width **132.** The terminal top width **130** may be greater than the terminal stalk width **132.** The terminal top width **130** may be greater than three times the terminal stalk width **132.** Alternatively, the terminal top width **130** may be greater than twice the terminal stalk width **132** or greater than 1.25 times the terminal stalk width **132.** Further, the orifice **122** has an orifice width **134** measured parallel to the terminal stalk width **132** and the terminal top width **130.** The orifice width **134** may be less than the terminal top width **130.** The orifice width **134** may be greater than the terminal stalk width **132,** or alternatively may be equal to or less than the terminal stalk width **132.**

The configuration illustrated in **FIGs. 11** and **12** may provide for easier and quicker attachment of the signaling device **110** to the garment. Additionally, since the terminal top **126** is held like a button in a button hole, no additional attachment mechanisms may be needed to attach the signaling device **110** to the garment. The terminal stalk **128** may be ridgely attached to the signaling device **110,** like a ball on the top of a pole.

The terminal stalk **128** may be attached to the signaling device **110** such that the terminal top **126** may be moved away from the signaling device **110** for attachment and detachment and moved toward the signaling device **110** for use. The terminal stalk **128** and the terminal top **126** may be formed from ridged materials. Alternatively, the terminal stalk **128** or the terminal top **126** may be formed from pliable materials depending upon the specific design of the chassis **32** and signaling device **110.**

**FIG. 13** illustrates a signaling device receptacle **140** which defines a pocket having an open edge **166.** The signaling device may pass through the open edge **166** to enter the receptacle **140.** The receptacle **140** defining the pocket may be formed from a single piece of material folded over upon it self to create the pocket. Alternatively, the receptacle **140** defining the pocket may be formed from two or more pieces of material joined together to create the pocket. The open edge **166** of the receptacle **140** may be elasticized to aid in maintaining the signaling device **110** in the receptacle **140.** The open edge **166** may be elasticized by a waist elastic member component **54** or **56.** Alternatively, the open edge **166** may be elasticized by one of the materials forming the pocket.
The open edge **166** may have a width **168,** and the pocket may have a maximum width **170** parallel to the open edge. The open edge width **168** before stretching may be less than the maximum width **170;** alternatively, the open edge width **168** before stretching may be less than 90%, or 75% of the maximum width **170.** The maximum width **170** may be determined by the specific signaling device, being slightly smaller or slightly larger than signaling device. This feature may act to push the signaling device **110** into the pocket during use, thereby reducing the chance the signaling device **110** may fall out and become inoperative.

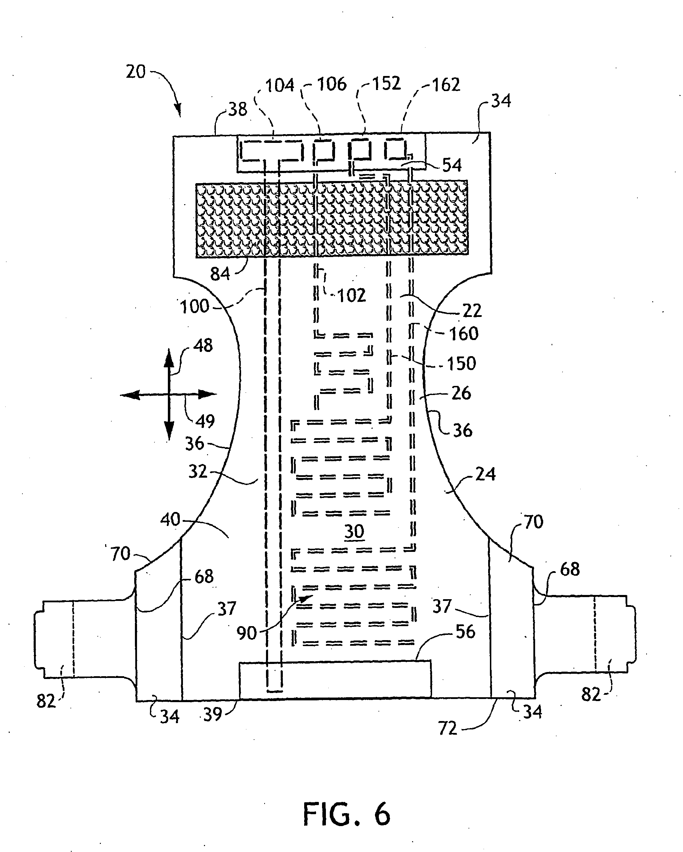

Referring to **Fig. 6****,** still another embodiment of an absorbent article **20** made in accordance with the present invention is shown. In the embodiment illustrated in **Fig. 6****,** in addition to the first conductive element **100** and the second conductive element **102,** the absorbent article includes a third conductive element **150** and a fourth conductive element **160.** As illustrated, the first conductive element **100** extends the entire length of the absorbent article. The second conductive element **102,** however, is located primarily within the front region of the chassis. The third conductive element **150** passes through the front region and is primarily located in the crotch region of the chassis.

Finally, the fourth conductive element **160** extends through the front region and the crotch region and then is primarily located in the back region of the chassis.

Similar to the conductive elements **100** and **102,** the third conductive element **150** may be connected to a third conductive pad member **152,** while the fourth conductive element **160** may be connected to a fourth conductive pad member **162.**

In the embodiment illustrated in **Fig. 6****,** the additional conductive elements are used for not only indicating the presence of a body fluid but also indicating the location of the body fluid. For instance, if the body fluid is located in the front region, the circuit between the first conductive element **100** and the second conductive element **102** is closed. If the body fluid is contained in the crotch region, on the other hand, the circuit formed between the third conductive element **150** and the first conductive element **100** becomes closed. In similar fashion, if the body fluid is located in the back region, the circuit becomes closed between the fourth conductive element **160** and the first conductive element **100.**

The embodiment illustrated in **Fig. 6** is intended to be placed in conjunction with a signaling device that includes four corresponding terminals for each of the four conductive pad members. The signaling device can then be configured to not only indicate when a body fluid is present but also indicate which circuits are closed, thus indicating the location of the body fluid in addition to its mere presence.

The remaining materials used to form the absorbent article **20** that surround the signaling device receptacle **140** may vary depending upon the particular application and the particular product being produced.

The outer cover **40,** for instance, may be breathable and/or may be liquid impermeable. The outer cover **40** may be constructed of a single layer, multiple layers, laminates, spunbond fabrics, films, meltblown fabrics, elastic netting, microporous webs, bonded card webs or foams provided by elastomeric or polymeric materials. The outer cover **40,** for instance, can be a single layer of a liquid impermeable material, or alternatively can be a multi-layered laminate structure in which at least one of the layers is liquid impermeable. In other embodiments, however, it should be understood that the outer cover may be liquid permeable. In this embodiment, for instance, the absorbent article may contain an interior liquid barrier layer.

For instance, the outer cover **40** can include a liquid permeable outer layer and a liquid impermeable inner layer that are suitably joined together by a laminate adhesive, ultrasonic bonds, thermal bonds, or the like. Suitable laminate adhesives, which can be applied continuously or intermittently as beads, a spray, parallel swirls, or the like, can be obtained from Bostik Findley Adhesives, Inc., of Wauwatosa, Wisconsin, U.S.A., or from National Starch and Chemical Company, Bridgewater, New Jersey U.S.A. The liquid permeable outer layer can be any suitable material and is desirably one that provides a generally cloth-like texture. One example of such a material is a 20 gsm (grams per square meter) spunbond polypropylene nonwoven web. The outer layer may also be made of those materials of which the liquid permeable bodyside liner **42** is made.

The inner layer of the outer cover **40** can be both liquid and vapor impermeable, or it may be liquid impermeable and vapor permeable. The inner layer can be manufactured from a thin plastic film, although other flexible liquid impermeable materials may also be used. The inner layer, or the liquid impermeable outer cover **40** when a single layer, prevents waste material from wetting articles, such as bed sheets and clothing, as well as the wearer and caregiver. A suitable liquid impermeable film for use as a liquid impermeable inner layer, or a single layer liquid impermeable outer cover **40,** is a 0.02 millimeter polyethylene film commercially available from Pliant Corporation of Schaumburg, Illinois, U.S.A.

The bodyside liner **42** is suitably compliant, soft-feeling, and non-irritating to the wearer's skin. The bodyside liner **42** is also sufficiently liquid permeable to permit liquid body exudates to readily penetrate through its thickness to the absorbent structure **44.** A suitable bodyside liner **42** may be manufactured from a wide selection of web materials, such as porous foams, reticulated foams, apertured plastic films, woven and non-woven webs, or a combination of any such materials. For example, the bodyside liner **42** may include a meltblown web, a spunbonded web, or a bonded-carded-web composed of natural fibers, synthetic fibers or combinations thereof. The bodyside liner **42** may be composed of a substantially hydrophobic material, and the hydrophobic material may optionally be treated with a surfactant or otherwise processed to impart a desired level of wettability and hydrophilicity.

The absorbent structure **44** may be disposed between the outer cover **40** and the bodyside liner **42.** The absorbent structure **44** can be any structure or combination of components which are generally compressible, conformable, non-irritating to a wearer's skin, and capable of absorbing and retaining liquids and certain body wastes. For example, the absorbent structure **44** may include an absorbent web material of cellulosic fibers (e.g., wood pulp fibers), other natural fibers, synthetic fibers, woven or nonwoven sheets, scrim netting or other stabilizing structures, superabsorbent material, binder materials, surfactants, selected hydrophobic materials, pigments, lotions, odor control agents or the like, as well as combinations thereof. In a particular aspect, the absorbent web material is a matrix of cellulosic fluff and superabsorbent hydrogel-forming particles. The cellulosic fluff may include a blend of wood pulp fluff. One preferred type of fluff is identified with the trade designation CR 1654, available from Bowater of Greenville, South Carolina, USA, and is a bleached, highly absorbent sulfate wood pulp containing primarily southern soft wood fibers. The absorbent materials may be formed into a web structure by employing various conventional methods and techniques. For example, the absorbent web may be formed with a dry-forming technique, an air forming technique, a wet-forming technique, a foam-forming technique, or the like, as well as combinations thereof. Methods and apparatus for carrying out such techniques are well known in the art. Furthermore, the absorbent structure may itself encompass multiple layers in the Z direction. Such multiple layers may take advantage of differences in absorbency capacity, such as by placing a lower capacity absorbent material layer closer to the liner **42** and a higher capacity absorbent material closer to the outer cover layer **40.** Likewise, discrete portions of an absorbent single-layered structure may encompass higher capacity absorbents, and other discrete portions of the structure may encompass lower capacity absorbents.

As a general rule, the superabsorbent material is present in the absorbent web in an amount of from about 0 to about 90 weight percent based on total weight of the web. The web may have a density within the range of about 0.10 to about 0.60 grams per cubic centimeter.

Superabsorbent materials are well known in the art and can be selected from natural, synthetic, and modified natural polymers and materials. The superabsorbent materials can be inorganic materials, such as silica gels, or organic compounds, such as crosslinked polymers. Typically, a superabsorbent material is capable of absorbing at least about 10 times its weight in liquid, and desirably is capable of absorbing more than about 25 times its weight in liquid. Suitable superabsorbent materials are readily available from various suppliers. For example, SXM 9394, and Favor 9543 superabsorbents are available from DeGussa Superabsorbers.

After being formed or cut into a desired shape, the absorbent web material may be wrapped or encompassed by a suitable tissue or meltblown web or the like wrap sheet that aids in maintaining the integrity and shape of the absorbent structure **44.**

The absorbent web material may also be a coform material. The term "coform material" generally refers to composite materials comprising a mixture or stabilized matrix of thermoplastic fibers and a second non-thermoplastic material. As an example, conform materials may be made by a process in which at least one meltblown die head is arranged near a chute through which other materials are added to the web while it is forming. Such other materials may include, but are not limited to, fibrous organic materials such as woody or non-woody pulp such as cotton, rayon, recycled paper, pulp fluff and also superabsorbent particles, inorganic absorbent materials, treated polymeric staple fibers and the like. Any of a variety of synthetic polymers may be utilized as the melt-spun component of the coform material. For instance, in certain aspects, thermoplastic polymers can be utilized. Some examples of suitable thermoplastics that can be utilized include polyolefins, such as polyethylene, polypropylene, polybutylene and the like; polyamides; and polyesters. In one aspect, the thermoplastic polymer is polypropylene. Some examples of such coform materials are disclosed in U.S. Patent Nos. 4,100,324 to Anderson, et al.; 5,284,703 to Everhart, et al.; and 5,350,624 to Georger, et al.

These and other modifications and variations to the present invention may be practiced by those of ordinary skill in the art, without departing from the scope of the present invention, which is set forth in the appended claims. In addition, it should be understood that aspects of the various embodiments may be interchanged both in whole or in part. Furthermore, those of ordinary skill in the art will appreciate that the foregoing description is by way of example only, and is not intended to limit the invention so further described in such appended claims.

## Claims

1. A garment (20) comprising:
a chassis (32) comprising an outer cover (40) having an interior surface and an exterior surface, the chassis including a crotch region (26) positioned in between a front region (22) and a back region (24), the front region and the back region jointly defining a waist region;
first and second conductive elements (100, 102) contained in the chassis, the conductive elements located in at least the waist region, the first and second conductive elements forming part of a circuit; and
a signaling device (110) including at least one first terminal and at least one second terminal (116, 118), the first and second terminals adapted to operatively connect to the first and second conductive elements;
wherein the first and second terminals operatively connect to the first and second conductive elements (100, 102) through at least one orifice (122) in the outer cover, and **characterized in that** the orifice (122) is defined by an orifice perimeter, the orifice perimeter (124) being elastic.

2. The garment of claim 1 wherein the first terminal and the second terminals (116, 118) connect to the first and second conductive elements (100, 102) through a single orifice (122); or wherein the first terminal (116) connects to the first or second conductive element (100, 102) through a first orifice and the second terminal (118) connects to the first or second conductive element (100, 102) through a second orifice.

3. The garment of claim 1 further comprising a first conductive pad member (104) electrically connected to the first conductive element (100) and a second conductive pad member (106) electrically connected to the second conductive element (102), the conductive pad members each being configured to electrically connect the conductive elements to the terminals.

4. The garment of claim 1, 2 or 3 further comprising an absorbent structure (44) positioned adjacent the interior surface of the outer cover (40), the outer cover preferably comprising multiple layers.

5. The garment of any preceding claim wherein the signaling device produces an audible signal, a visible signal, or vibrates upon completion of the circuit; or wherein the signaling device (110) comprises a transmitter (112) containing the first terminal and the second terminal that are electrically connected to the first and second conductive elements, and a receiver (114), wherein, the transmitter sends a wireless signal to the receiver which produces an audible or visible signal to a user when the signaling device (110) is activated.

6. The garment of any preceding claim wherein the first terminal (116) comprise a terminal top (126) and a terminal stalk (128), the terminal top having a terminal top width, the terminal stalk having a terminal stalk width (132), the terminal top width (130) being greater than the terminal stalk width, preferably is greater than twice the terminal stalk width.

7. The garment of any preceding claim wherein the first and second conductive elements (100, 102) extend from the waist region to the crotch region without intersecting.

8. The garment of any preceding claim wherein the first and second conductive elements (100, 102) form an open circuit and wherein, when a body fluid is present between the first and second conductive elements, the body fluid closes the circuit to activate the signaling device.

9. The garment of claim 1 wherein the chassis includes a signaling device receptacle (140) adapted to contain the signaling device, the receptacle defining a pocket having an open edge, the open edge being elasticized.

10. The garment of any of claims 1 to 8 wherein the chassis includes a signaling device receptacle (140) adapted to contain the signaling device, the receptacle (140) defining a pocket having an open edge (166) having an open edge width (168) and the pocket having a maximum width parallel to the open edge, the open edge width being less than the maximum width, preferably less than 90 percent the maximum width.

11. The garment of claim 1 wherein the chassis includes a signaling device receptacle (140) adapted to contain the signaling device, the receptacle comprising a first material and a second material joined to the first material, the first conductive element (100) connected to the first material, the second conductive element (102) connected to the second material.

12. The garment of any preceding claim wherein the first terminal (116) has an area, the second terminal (118) has an area, the orifice has an orifice area (122) and the orifice area is greater than a combined area of the first terminal area and the second terminal area.

13. The garment of claim 6 wherein the terminal top is adapted to be moved away from a main body of the signaling device during attachment and detachment and adapted to be moved toward the main body of the signaling device during for use.

## Patentansprüche

1. Kleidungsstück (20) umfassend:
einen Rahmen (32), der eine äußere Deckschicht (40) umfasst, die eine innere Oberfläche und eine äußere Oberfläche aufweist, wobei der Rahmen einen Schrittbereich (26) beinhaltet, der zwischen einem Vorderbereich (22) und einem Hinterbereich (24) angeordnet ist, wobei der Vorderbereich und der Hinterbereich miteinander einen Taillenbereich definieren;
erste und zweite leitende Elemente (100, 102), die im Rahmen enthalten sind, wobei die leitenden Elemente zumindest in dem Taillenbereich angeordnet sind, wobei die ersten und zweiten leitenden Elemente Teile eines Schaltkreises bilden; und
eine Signalvorrichtung (110), welche mindestens einen ersten Anschluss und mindestens einen zweiten Anschluss (116, 118) beinhaltet, wobei der erste und zweite Anschluss eingerichtet sind, um mit dem ersten und zweiten leitenden Element wirkverbunden zu sein;
wobei sich der erste und zweite Anschluss wirksam mit dem ersten und zweiten leitenden Element (100, 102) durch mindestens eine Öffnung (122) in der äußeren Deckschicht verbinden, und **dadurch gekennzeichnet, dass** die Öffnung (122) durch einen Öffnungsperimeter definiert ist, wobei der Öffnungsperimeter (124) elastisch ist.

2. Das Kleidungsstück gemäß Anspruch 1, wobei sich der erste Anschluss und der zweite Anschluss (116, 118) mit dem ersten und zweiten leitenden Element (100, 102) durch eine einzelne Öffnung (122) verbinden; oder wobei sich der erste Anschluss (116) mit dem ersten oder zweiten leitenden Element (100, 102) durch eine erste Öffnung verbindet, und sich der zweite Anschluss (118) mit dem ersten oder zweiten leitenden Element (100, 102) durch eine zweite Öffnung verbindet.

3. Das Kleidungsstück gemäß Anspruch 1, welches des Weiteren ein erstes leitendes Padteil (104) umfasst, welches mit dem ersten leitenden Element (100) elektrisch verbunden ist, und ein zweites leitendes Padteil (108) umfasst, welches mit dem zweiten leitenden Element (102) elektrisch verbunden ist, wobei jedes der leitenden Padteile eingerichtet ist, die leitenden Elemente mit den Anschlüssen elektrisch zu verbinden.

4. Das Kleidungsstück gemäß Anspruch 1, 2 oder 3, welches des Weiteren eine absorbierende Struktur (44) umfasst, welche angrenzend an die innere Oberfläche der äußeren Deckschicht (40) angeordnet ist, wobei die äußere Deckschicht vorzugsweise mehrere Schichten umfasst.

5. Das Kleidungsstück gemäß einem der vorherigen Ansprüche, wobei die Signalvorrichtung bei Schließen des Schaltkreises ein akustisches Signal oder ein sichtbares Signal erzeugt oder vibriert; oder wobei die Signalvorrichtung (110) einen Transmitter (112) umfasst, der den ersten Anschluss und den zweiten Anschluss enthält, die elektrisch mit dem ersten und zweiten leitenden Element verbunden sind, und einen Empfänger (114), wobei der Transmitter ein kabelloses Signal an den Empfänger sendet, welches ein akustisches oder sichtbares Signal an einen Benutzer erzeugt, wenn die Signalvorrichtung (110) aktiviert wird.

6. Das Kleidungsstück gemäß einem der vorherigen Ansprüche, wobei der erste Anschluss (116) eine Anschlussoberseite (126) und einen Anschlussstiel (128) umfasst, wobei die Anschlussoberseite eine Anschlussoberseitenbreite aufweist, wobei der Anschlussstiel eine Anschlussstielbreite (132) aufweist, wobei die Anschlussoberseitenbreite (130) größer ist als die Anschlussstielbreite, vorzugsweise mehr als zweimal größer als die Anschlussstielbreite.

7. Das Kleidungsstück gemäß einem der vorherigen Ansprüche, wobei sich das erste und zweite leitende Element (100, 102) von dem Taillenbereich bis zum Schrittbereich ohne Überschneiden erstrecken.

8. Das Kleidungsstück gemäß einem der vorherigen Ansprüche, wobei das erste und zweite leitende Element (100, 102) einen offenen Schaltkreis bilden, und wobei, wenn eine Körperflüssigkeit zwischen dem ersten und zweiten leitenden Element vorhanden ist, die Körperflüssigkeit den Schaltkreis schließt, um die Signalvorrichtung zu aktivieren.

9. Das Kleidungsstück gemäß Anspruch 1, wobei der Rahmen eine Signalvorrichtungsaufnahme (140) beinhaltet, welche eingerichtet ist, die Signalvorrichtung zu enthalten, wobei die Aufnahme eine Tasche definiert, welche eine offene Kante aufweist, wobei die offene Kante elastisch gemacht ist.

10. Das Kleidungsstück gemäß einem der Ansprüche 1 bis 8, wobei der Rahmen eine Signalvorrichtungsaufnahme (140) beinhaltet, welche eingerichtet ist, die Signalvorrichtung zu enthalten, wobei die Aufnahme (140) eine Tasche definiert, welche eine offene Kante (166) aufweist, welche eine Breite der offenen Kanten (168) aufweist, und wobei die Tasche eine Maximalbreite parallel zu der offenen Kante aufweist, wobei die Breite der offenen Kante geringer ist als die Maximalbreite, vorzugsweise weniger als 90 Prozent der Maximalbreite.

11. Das Kleidungsstück gemäß Anspruch 1, wobei der Rahmen eine Signalvorrichtungsaufnahme (140) beinhaltet, welche eingerichtet ist, die Signalvorrichtung zu enthalten, wobei die Aufnahme ein erstes Material und ein zweites Material umfasst, welches mit dem ersten Material verbunden ist, wobei das erste leitende Element (110) mit dem ersten Material verbunden ist, wobei das zweite leitende Element (102) mit dem zweiten Material verbunden ist.

12. Das Kleidungsstück gemäß einem der vorherigen Ansprüche, wobei der erste Anschluss (116) einen Bereich aufweist, wobei der zweite Anschluss (118) einen Bereich aufweist, wobei die Öffnung einen Öffnungsbereich (122) aufweist, und wobei der Öffnungsbereich größer ist als ein kombinierter Bereich des ersten Anschlussbereichs und des zweiten Anschlussbereichs.

13. Das Kleidungsstück gemäß Anspruch 6, wobei die Anschlussoberseite eingerichtet ist, um während des Befestigens und Lösens von einem Hauptkörper der Signalvorrichtung weg bewegt zu werden, und eingerichtet ist während der Verwendung in Richtung des Hauptkörpers der Signalvorrichtung bewegt zu werden.

## Revendications

1. Vêtement (20) comprenant :
un châssis (32) comprenant une enveloppe extérieure (40) ayant une surface intérieure et une surface extérieure, le châssis comportant une région d'entrejambe (26) positionnée entre une région avant (22) et une région arrière (24), la région avant et la région arrière définissant conjointement une région de ceinture ;
des premier et second éléments conducteurs (100, 102) contenus dans le châssis, les éléments conducteurs étant situés au moins dans la région de ceinture, les premier et second éléments conducteurs faisant partie d'un circuit ; et
un dispositif de signalisation (110) comportant au moins une première borne et au moins une seconde borne (116, 118), les première et seconde bornes étant aptes à se connecter fonctionnellement aux premier et second éléments conducteurs ;
dans lequel les première et seconde bornes se connectent fonctionnellement aux premier et second éléments conducteurs (100, 102) au travers d'au moins un orifice (122) de l'enveloppe extérieure, et
**caractérisé en ce que** l'orifice (122) est défini par un périmètre d'orifice, le périmètre d'orifice (124) étant élastique.

2. Vêtement selon la revendication 1, dans lequel la première borne et la seconde borne (116, 118) se connectent aux premier et second éléments conducteurs (100, 102) au travers d'un orifice unique (122) ; ou dans lequel la première borne (116) se connecte au premier ou second élément conducteur (100, 102) au travers d'un premier orifice et la seconde borne (118) se connecte au premier ou second élément conducteur (100, 102) au travers d'un second orifice.

3. Vêtement selon la revendication 1, comprenant un premier élément de tampon (104) électriquement connecté au, premier élément conducteur (100) et un second élément de tampon (106) électriquement connecté au second élément conducteur (102), les éléments de tampon conducteurs étant chacun configurés pour connecter les éléments conducteurs aux bornes.

4. Vêtement selon la revendication 1, 2 ou 3, comprenant en outre une structure absorbante (44) positionnée de façon adjacente à la surface intérieure de l'enveloppe extérieure (40), l'enveloppe extérieure comprenant de préférence de multiples couches.

5. Vêtement selon l'une quelconque des revendications précédentes, dans lequel le dispositif de signalisation produit un signal audible, un signal visible, ou vibre lors de la fermeture du circuit ; ou dans lequel le dispositif de signalisation (110) comprend un émetteur (112) contenant la première borne et la seconde borne qui sont électriquement connectées aux premier et second éléments conducteurs, et un récepteur (114), dans lequel l'émetteur envoie un signal sans fil au récepteur qui produit un signal audible ou visible destiné à un utilisateur lorsque le dispositif de signalisation (110) est activé.

6. Vêtement selon l'une quelconque des revendications précédentes, dans lequel la première borne (116) comprend une extrémité supérieure de borne (126) et une tige de borne (128), l'extrémité supérieure de borne ayant une largeur d'extrémité supérieure de borne, la tige de borne ayant une largeur de tige de borne (132), la largeur d'extrémité supérieure de borne (130) étant supérieure à la largeur de tige de borne et étant de préférence deux fois supérieure à la largeur de tige de borne.

7. Vêtement selon l'une quelconque des revendications précédentes, dans lequel les premier et second éléments conducteurs (100, 102) se prolongent de la région de ceinture vers la région d'entrejambe sans intersection.

8. Vêtement selon l'une quelconque des revendications précédentes, dans lequel les premier et second éléments conducteurs (100, 102) forment un circuit ouvert et dans lequel, lorsqu'un fluide corporel est présent entre les premier et second éléments conducteurs, le fluide corporel ferme le circuit en activant le dispositif de signalisation.

9. Vêtement selon la revendication 1, dans lequel le châssis comporte un réceptacle pour dispositif de signalisation (140) apte à contenir le dispositif de signalisation, le réceptacle définissant une poche ayant un bord ouvert, le bord ouvert étant élastifié.

10. Vêtement selon l'une quelconque des revendications 1 à 8, dans lequel le châssis comprend un réceptacle pour dispositif de signalisation (140) apte à contenir le dispositif de signalisation, le réceptacle (140) définissant une poche ayant un bord ouvert (166) ayant une largeur de bord ouvert (168) et la poche ayant une largeur maximale parallèlement au bord ouvert, la largeur du bord ouvert étant inférieure à la largeur maximale, et de préférence, inférieure à 90 % de la largeur maximale.

11. Vêtement selon la revendication 1, dans lequel le châssis comprend un réceptacle pour dispositif de signalisation (140) apte à contenir le dispositif de signalisation, le réceptacle comprenant un premier matériau et un second matériau joint au premier matériau, le premier élément conducteur (100) étant connecté au premier matériau, et le second élément conducteur (102) étant connecté au second matériau.

12. Vêtement selon l'une quelconque des revendications précédentes, dans lequel la première borne (116) a une superficie, la seconde borne (118) a une superficie, l'orifice a une superficie d'orifice (122) et la superficie d'orifice est supérieure à une superficie combinée de la superficie de la première borne et de la superficie de la seconde borne.

13. Vêtement selon la revendication 6, dans lequel la partie supérieure de la borne est apte à être éloignée d'un corps principal du dispositif de signalisation pendant l'attachement et le détachement et est apte à être rapprochée du corps principal du dispositif de signalisation pendant l'utilisation.
